# EUROPEAN PATENT SPECIFICATION

(11) **EP 3 648 825 B1**
(45) Date of publication and mention of the grant of the patent: **29.09.2021**
(21) Application number: 18740522.0
(22) Date of filing: 28.06.2018
(51) Int. Cl.: A61M 16/08

(54) **NASAL CONTINUOUS POSITIVE AIRWAY PRESSURE DEVICE AND SYSTEM**
NASALE VORRICHTUNG UND SYSTEM FÜR KONTINUIERLICHEN POSITIVEN ATEMWEGSDRUCK
SYSTÈME ET DISPOSITIF À PRESSION POSITIVE CONTINUE POUR VOIES RESPIRATOIRES NASALES

(30) Priority: 03.07.2017 DK PA201700397
(43) Date of publication of application: 13.05.2020
(73) Proprietor: INNO3 APS, 8410 Rønde (DK)
(72) Inventor: RASMUSSEN, Ann-Christina, Lykke, 8410 Rønde (DK); MOTZFELDT, Michael, 8410 Rønde (DK)
(74) Representative: Larsen & Birkeholm A/S
(86) International application number: PCT/EP2018/067430
(87) International publication number: WO 2019/007805

(56) References cited:
- EP-A1- 0 467 362
- US-A- 4 463 755
- US-A- 5 404 873
- US-A1- 2010 132 706

## Description

### Technical field of the invention

The present invention relates to the field of continuous positive airway pressure (CPAP) therapy, more specifically to nasal continuous positive airway pressure (nCPAP) systems.

### Background of the invention

Continuous positive airway pressure (CPAP) therapy has been employed for many years to treat patients experiencing respiratory difficulties and/or insufficiencies. More recently, CPAP therapy has been advanced as being useful in assisting patients with under-developed lungs (especially infants, such as neonates), by preventing lung collapse during exhalation and assisting lung expansion during inhalation.

In general terms, CPAP therapy entails the continuous transmission of positive pressure into the lungs of a spontaneously breathing patient throughout the respiratory cycle. CPAP can be delivered to the patient using a variety of patient interface devices, for example an endotracheal tube. With infants, however, it is more desirable to employ a less invasive patient interface device, in particular one that interfaces directly or indirectly with the nasal airways via the patient's nares (e.g., mask or nasal prongs). Such systems are commonly referred to as nasal continuous positive airway pressure (nCPAP) systems.

In theory, the CPAP system should deliver a constant, stable pressure to the patient's airways. With conventional, ventilator-based CPAP devices, a relative constant and continuous flow of gas (e.g., air, oxygen gas, etc.) is delivered into the patient's airways, with this airflow creating a pressure within the patient's lungs via a restriction placed on outflow from the patient. Unfortunately, this continuous flow can have an adverse effect on the patient's respiratory synchrony. More particularly, the patient is required to exhale against the incoming gas, thus increasing the patient's work of breathing. For an infant with under developed lungs, even a slight increase in the required work of breathing may render the CPAP system in question impractical.

More recently, nCPAP systems have been developed that incorporate a variable flow concept in combination with separate channels for inspiratory and expiratory gas to and from the patient. When the patient inhales, the incoming gas takes the path of least resistance and is directed to the patient's airways. Upon expiration, the gas again takes the path of least resistance and goes out an exhalation or exhaust tube, thus reducing resistance during the expiratory phase. High levels of noise are a stressful factor that can have tremendous effects on a neonate's recovery. While highly specialized treatments can greatly improve a neonate's medical condition, physical surroundings are rarely regarded as influential in his or her care. The crowded neonatal intensive care units are usually packed with nurses, equipment and lots of noise that affect the neonates" stress levels. These effects are clearly noticeable in neonates' monitors that show how a lot of noise can agitate their fragile hearts. In addition, these loud, crowded environments are also not particularly family friendly, and parents often do not feel comfortable about spending time there (Praskach, Ana, "Nature, daylight and sound: A sensible environment for the families, staff and patients of neonatal intensive care units" (2009). Graduate Theses and Dissertations.). A general problem with current nCPAP systems is that noise is generated close to the head of the patient.

US4463755 A and EP0467362 A1, both disclose breathing circuits comprising an elbow connected to a mask, and a coaxial tube comprising an inner tube constituting an inhalation circuit and an outer tube encircling the inner tube, and defining an exhalation circuit, wherein the lumens of the inner tube and the outer tube are in fluid communication with a lumen of the elbow.

### Object of the Invention

The objective of the present invention is to provide an nCPAP system with a reduced noise generation close to the head of the patient.

### Description of the Invention

The present invention is defined in the appended independent claim 1. Preferred embodiments are matter of the dependent claims. The present invention relates to a nasal continuous positive airway pressure device comprising:
- an oxygen gas supply;
- a neck region connected to a mask or nasal prong;
- an elongated flexible tube comprising co-axial inner and outer tubes, and
wherein the inner and outer tubes are spaced to define a first lumen within the outer tube, and wherein a second lumen is defined by the inner tube wall alone; wherein the second lumen is connected to the oxygen gas supply at the distal end of the elongated flexible tube, and to the neck region at the proximal end of the elongated flexible tube; and wherein the first lumen is connected to the neck region at the proximal end of the elongated flexible tube. This configuration allows for a very compact nasal continuous positive airway pressure device that appears less dangerous to the parents of the neonate. More importantly, this configuration reduces the stress of the neonate by removing the exhaust air and noise from the neonate 's face.

Preferably, the length of the outer tube is at least 20 cm, such as within the range of 20-200 cm, e.g. within the range of 25-150 cm, such as within the range of 30-125 cm, e.g. within the range of 35-100 cm, such as within the range of 40-90 cm, e.g. within the range of 45-80 cm, such as within the range of 50-70 cm, e.g. within the range of 55-65 cm. This configuration reduces the stress of the neonate by removing the exhaust air and noise from the neonate's face.

Further according to the invention, the neck region comprises:
- a tube part connected to the proximal end of the inner tube, and comprising a lumen in fluid communication with the second lumen; and
- a cone part connected to the proximal end of the outer tube, and comprising a lumen in fluid communication with the first lumen; wherein the cone part is positioned around at least a part of the tube part;
wherein the lumen of the tube part is in fluid communication with the lumen of the cone part through one or more apertures in the wall of the tube part. This configuration decreases the patient's work of breathing during exhalation.

In one or more embodiments, the neck region comprises a gas pressure port adapted for being connected to means for measuring the gas pressure within the neck region. This configuration allows for control of the oxygen supply to the patient.

In one or more embodiments, the tube part comprises a gas pressure port adapted for being connected to means for measuring the gas pressure within the tube part.

In one or more embodiments, the tube part comprises a straight tube section, and a bend tube section.

In one or more embodiments, the tube part comprises a straight tube section, and a bend tube section, and wherein the one or more apertures are positioned in the straight tube section.

In one or more embodiments, the tube part comprises a straight tube section, and a bend tube section, and wherein the one or more apertures are positioned in the straight tube section, and wherein the gas pressure port is positioned in the bend tube section.

In one or more embodiments, the one or more apertures are configured as an elongate recess.

In one or more embodiments, one or more apertures are circular.

In one or more embodiments, lumen of the tube part is in fluid communication with the lumen of the cone part through a plurality of apertures in the wall of the tube part.

As used in the specification and the appended claims, the singular forms "a", "an", and "the" include plural referents unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about", it will be understood that the particular value forms another embodiment.

It should be noted that embodiments and features described in the context of one of the aspects of the present invention also apply to the other aspects of the invention.

### Brief description of the figures

Figure 1 is a perspective view of a nasal continuous positive airway pressure device in accordance with various embodiments of the invention;

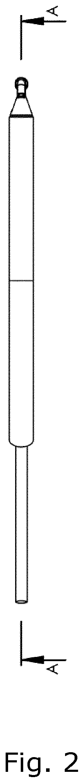

Figure 2 is a top view of a nasal continuous positive airway pressure device in accordance with various embodiments of the invention;
Figure 3 is the cross-sectional view A-A of Figure 2, shown as a side view; and
Figures 4-5 are a detailed view B of Figure 3.

### References

- 100: Nasal continuous positive airway pressure device
- 200: Neck region
- 210: Tube part
- 212: Aperture
- 214: Lumen
- 216: Gas pressure port
- 218: Straight tube section
- 219: Bend tube section
- 220: Cone part
- 222: Lumen
- 300: Flexible tube
- 302: Proximal end
- 304: Distal end
- 310: Inner tube
- 312: Distal end
- 320: Outer tube
- 322: Distal end
- 330: First lumen
- 340: Second lumen

### Detailed Description of the Invention

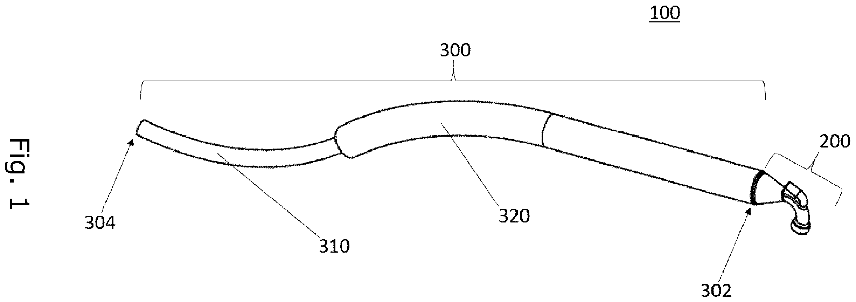

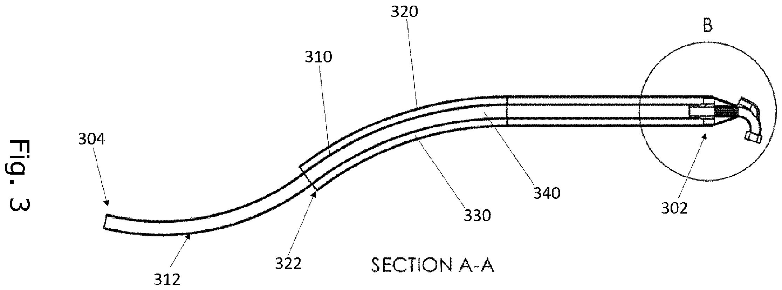

Figure 1 is a perspective view of a nasal continuous positive airway pressure device in accordance with various embodiments of the invention. The nasal continuous positive airway pressure device 100 comprises a neck region 200 adapted for being connected to a mask or nasal prong (not shown); and an elongated flexible tube 300. The elongated flexible tube 300 comprises co-axial inner 310 and outer 320 tubes. The inner 310 and outer 320 tubes are spaced to define a first lumen 330 within the outer tube 320 (Figure 3). A second lumen 340 is defined by the inner tube wall alone. The second lumen 340 is configured to be connected to an oxygen gas supply at the distal end 304 of the elongated flexible tube 300, and to the neck region 200 at the proximal end 302 of the elongated flexible tube 300. The first lumen 330 is configured to be connected to the neck region 200 at the proximal end 302 of the elongated flexible tube 300.

The distal end 312 of the inner tube 310 extends out of the outer tube 320 at its distal end 322, such that the first lumen 330 is in fluid communication with the surrounding atmosphere at the distal end 322 of the outer tube 320 tube.

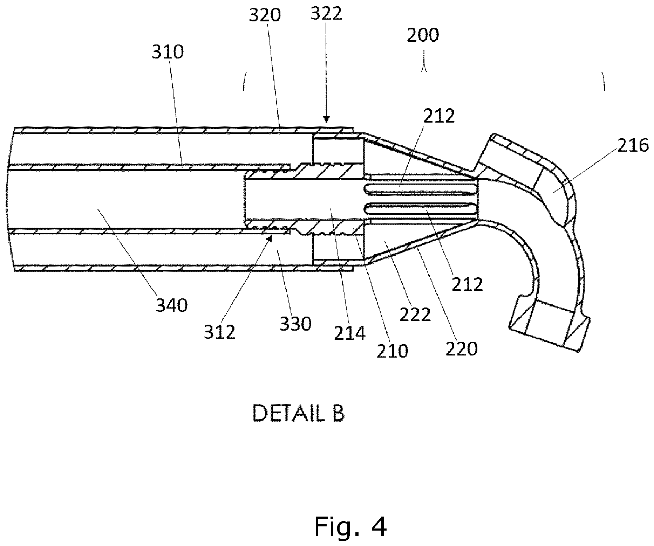

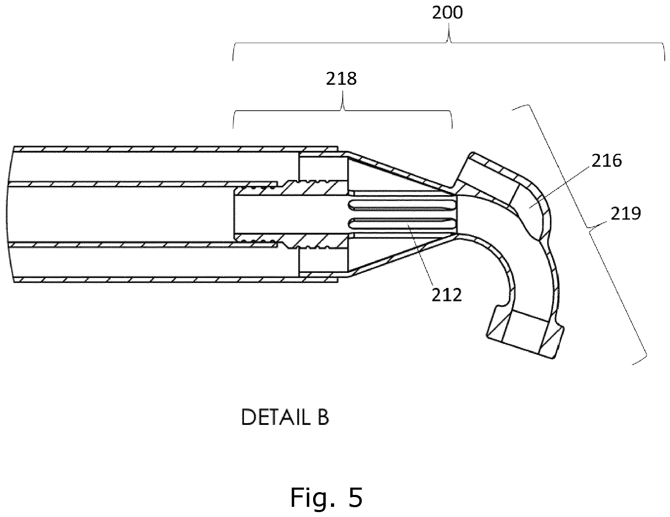

Figures 4-5 are a detailed view B of Figure 3. The neck region 200 comprises a tube part 210 adapted for being connected to the proximal end 312 of the inner tube 310 and comprising a lumen 214 in fluid communication with the second lumen 340. The neck region 200 also comprises a cone part 220 adapted for being connected to the proximal end 322 of the outer tube 320 and comprising a lumen 222 in fluid communication with the first lumen 330. The cone part 220 is positioned around a part of the tube part 210. The lumen 214 of the tube part 210 is in fluid communication with the lumen 222 of the cone part 220 through a plurality of apertures 212 in the wall of the tube part 210.

The tube part 210 comprises a straight tube section 218, and a bend tube section 219. A plurality of apertures 212 are positioned in the straight tube section 218. Furthermore, a gas pressure port 216 is positioned in the bend tube section 219.

## Claims

1. A nasal continuous positive airway pressure device (100) comprising:
- a neck region (200) connected to a mask or nasal prong; and
- an elongated flexible tube (300) comprising co-axial inner (310) and outer (320) tubes, and wherein the inner (310) and outer (320) tubes are spaced to define a first lumen (330) within the outer tube (320), and wherein a second lumen (340) is defined by the inner tube wall alone; and
- an oxygen gas supply;
**characterized in that** the second lumen (340) is connected to said oxygen gas supply at the distal end (304) of the elongated flexible tube system (300), and to said neck region (200) at the proximal end (302) of the elongated flexible tube (300); wherein the first lumen (330) is connected to the neck region (200) at the proximal end (302) of the elongated flexible tube (300); wherein the neck region (200) comprises:
- a tube part (210) connected to the proximal end (312) of the inner tube (310), and comprising a lumen (214) in fluid communication with the second lumen (340); and
- a cone part (220) connected to the proximal end (322) of the outer tube (320), and comprising a lumen (222) in fluid communication with the first lumen (330); wherein the cone part (220) is positioned around at least a part of the tube part (210);
wherein the lumen (214) of the tube part (210) is in fluid communication with the lumen (222) of the cone part (220) through one or more apertures (212) in the wall of the tube part (210).

2. A nasal continuous positive airway pressure device (100) according to claim 1, **characterized in that** the neck region (200) comprises a gas pressure port adapted for being connected to means for measuring the gas pressure within the neck region (200).

3. A nasal continuous positive airway pressure device (100) according to claim 2, **characterized in that** the tube part (210) comprises a gas pressure port (216) adapted for being connected to means for measuring the gas pressure within the tube part (210).

4. A nasal continuous positive airway pressure device (100) according to claim 3, **characterized in that** the tube part (210) comprises a straight tube section (218), and a bend tube section (219), and wherein the one or more apertures (212) are positioned in the straight tube section (218), and wherein the gas pressure port (216) is positioned in the bend tube section (219).

5. A nasal continuous positive airway pressure device (100) according to any one of the claims 1-4, **characterized in that** the one or more apertures (212) are configured as an elongate recess.

6. A nasal continuous positive airway pressure device (100) according to any one of the claims 1-4, **characterized in that** the one or more apertures (212) are circular.

7. A nasal continuous positive airway pressure device (100) according to any one of the claims 1-6, **characterized in that** the lumen (214) of the tube part (210) is in fluid communication with the lumen (222) of the cone part (220) through a plurality of apertures (212) in the wall of the tube part (210).

8. A nasal continuous positive airway pressure device (100) according to any one of the claims 1-7, **characterized in that** the length of the outer tube (320) is at least 20 cm.

## Patentansprüche

1. Nasale Vorrichtung (100) für kontinuierlichen positiven Atemwegsdruck, umfassend:
- eine Halsregion (200), die mit einer Maske oder einer Nasengabel verbunden ist; und
- einen länglichen flexiblen Schlauch (300), der einen inneren (310) und einen äußeren (320) Schlauch, die koaxial sind, umfasst, und wobei der innere (310) und der äußere (320) Schlauch so beabstandet sind, dass sie ein erstes Lumen (330) innerhalb des äußeren Schlauchs (320) definieren, und wobei ein zweites Lumen (340) allein durch die Wand des inneren Schlauchs definiert ist; und
- eine Sauerstoffgaszufuhr;
**dadurch gekennzeichnet, dass** das zweite Lumen (340) mit der Sauerstoffgaszufuhr an dem distalen Ende (304) des länglichen flexiblen Schlauchsystems (300) und mit der Halsregion (200) an dem proximalen Ende (302) des länglichen flexiblen Schlauchsystems (300) verbunden ist; wobei das erste Lumen (330) mit der Halsregion (200) an dem proximalen Ende (302) des länglichen flexiblen Schlauchsystems (300) verbunden ist; wobei die Halsregion (200) Folgendes umfasst:
- einen Schlauchabschnitt (210), der mit dem proximalen Ende (312) des inneren Schlauchs (310) verbunden ist und ein Lumen (214) in Fluidverbindung mit dem zweiten Lumen (340) umfasst; und
- einen Konusabschnitt (220), der mit dem proximalen Ende (322) des äußeren Schlauchs (320) verbunden ist und ein Lumen (222) in Fluidverbindung mit dem ersten Lumen (330) umfasst; wobei der Konusabschnitt (220) um mindestens einen Teil des Schlauchabschnitts (210) angeordnet ist;
wobei das Lumen (214) des Schlauchabschnitts (210) in Fluidverbindung mit dem Lumen (222) des Konusabschnitts (220) durch eine oder mehrere Öffnungen (212) in der Wand des Schlauchabschnitts (210) steht.

2. Nasale Vorrichtung (100) für kontinuierlichen positiven Atemwegsdruck nach Anspruch 1, **dadurch gekennzeichnet, dass** die Halsregion (200) einen Gasdruckanschluss umfasst, der so ausgelegt ist, dass er mit einer Einrichtung zum Messen des Gasdrucks innerhalb der Halsregion (200) verbunden werden kann.

3. Nasale Vorrichtung (100) für kontinuierlichen positiven Atemwegsdruck nach Anspruch 2, **dadurch gekennzeichnet, dass** der Schlauchabschnitt (210) einen Gasdruckanschluss (216) umfasst, der so ausgelegt ist, dass er mit einer Einrichtung zum Messen des Gasdrucks innerhalb Schlauchabschnitts (210) verbunden werden kann.

4. Nasale Vorrichtung (100) für kontinuierlichen positiven Atemwegsdruck nach Anspruch 3, **dadurch gekennzeichnet, dass** der Schlauchabschnitt (210) einen geraden Schlauchteil (218) und einen gebogenen Schlauchteil (219) umfasst, und wobei die eine oder die mehreren Öffnungen (212) in dem geraden Schlauchteil (218) angeordnet sind und wobei der Gasdruckanschluss (216) in dem gebogenen Schlauchteil (219) angeordnet ist.

5. Nasale Vorrichtung (100) für kontinuierlichen positiven Atemwegsdruck nach einem der Ansprüche 1-4, **dadurch gekennzeichnet, dass** die eine oder die mehreren Öffnungen (212) als eine längliche Aussparung konfiguriert sind.

6. Nasale Vorrichtung (100) für kontinuierlichen positiven Atemwegsdruck nach einem der Ansprüche 1-4, **dadurch gekennzeichnet, dass** die eine oder die mehreren Öffnungen (212) kreisförmig sind.

7. Nasale Vorrichtung (100) für kontinuierlichen positiven Atemwegsdruck nach einem der Ansprüche 1-6, **dadurch gekennzeichnet, dass** das Lumen (214) des Schlauchabschnitts (210) in Fluidverbindung mit dem Lumen (222) des Konusabschnitts (220) durch eine Vielzahl von Öffnungen (212) in der Wand des Schlauchabschnitts (210) steht.

8. Nasale Vorrichtung (100) für kontinuierlichen positiven Atemwegsdruck nach einem der Ansprüche 1-7, **dadurch gekennzeichnet, dass** die Länge des äußeren Schlauchs (320) mindestens 20 cm beträgt.

## Revendications

1. Dispositif à pression positive continue pour voies respiratoires nasales (100) comprenant :
- une région de col (200) raccordée à un masque ou une canule nasale ; et
- un tube flexible allongé (300) comprenant des tubes intérieur (310) et extérieur (320) co-axiaux, et dans lequel les tubes intérieur (310) et extérieur (320) sont espacés pour définir une première lumière (330) à l'intérieur du tube extérieur (320), et dans lequel une seconde lumière (340) est définie par la paroi du tube intérieur seule ; et
- une alimentation en oxygène gazeux ;
**caractérisé en ce que** la seconde lumière (340) est raccordée à ladite alimentation en oxygène gazeux au niveau de l'extrémité distale (304) du système de tube flexible allongé (300), et à ladite région de col (200) au niveau de l'extrémité proximale (302) du tube flexible allongé (300) ; dans lequel la première lumière (330) est raccordée à la région de col (200) au niveau de l'extrémité proximale (302) du tube flexible allongé (300) ; dans lequel la région de col (200) comprend :
- une partie tube (210) raccordée à l'extrémité proximale (312) du tube intérieur (310), et comprenant une lumière (214) en communication fluidique avec la seconde lumière (340) ; et
- une partie cône (220) raccordée à l'extrémité proximale (322) du tube extérieur (320), et comprenant une lumière (222) en communication fluidique avec la première lumière (330) ; dans lequel la partie cône (220) est positionnée autour d'au moins une partie de la partie tube (210) ;
dans lequel la lumière (214) de la partie tube (210) est en communication fluidique avec la lumière (222) de la partie cône (220) à travers une ou plusieurs ouvertures (212) dans la paroi de la partie tube (210).

2. Dispositif à pression positive continue pour voies respiratoires nasales (100) selon la revendication 1, **caractérisé en ce que** la région de col (200) comprend un port de pression gazeuse adapté à être raccordé à un moyen de mesure de la pression gazeuse à l'intérieur de la région de col (200).

3. Dispositif à pression positive continue pour voies respiratoires nasales (100) selon la revendication 2, **caractérisé en ce que** la partie tube (210) comprend un port de pression gazeuse (216) adapté à être raccordé à un moyen de mesure de la pression gazeuse à l'intérieur de la partie tube (210) .

4. Dispositif à pression positive continue pour voies respiratoires nasales (100) selon la revendication 3, **caractérisé en ce que** la partie tube (210) comprend une section tube linéaire (218), et une section tube incurvée (219), et dans lequel les unes ou plusieurs ouvertures (212) sont positionnées dans la section tube linéaire (218), et dans lequel le port de pression gazeuse (216) est positionné dans la section tube incurvée (219).

5. Dispositif à pression positive continue pour voies respiratoires nasales (100) selon l'une quelconque des revendications 1 à 4, **caractérisé en ce que** les une ou plusieurs ouvertures (212) sont configurées comme une cavité allongée.

6. Dispositif à pression positive continue pour voies respiratoires nasales (100) selon l'une quelconque des revendications 1 à 4, **caractérisé en ce que** les une ou plusieurs ouvertures (212) sont circulaires.

7. Dispositif à pression positive continue pour voies respiratoires nasales (100) selon l'une quelconque des revendications 1 à 6, **caractérisé en ce que** la lumière (214) de la partie tubulaire (210) est en communication fluidique avec la lumière (222) de la partie cône (220) à travers une pluralité d'ouvertures (212) dans la paroi de la partie tube (210).

8. Dispositif à pression positive continue pour voies respiratoires nasales (100) selon l'une quelconque des revendications 1 à 7, **caractérisé en ce que** la longueur du tube extérieur (320) est d'au moins 20 cm.
